Europäisches Patentamt

European Patent Office

Office européen des brevets

(11) Publication number: **0 520 672 A2**

# EUROPEAN PATENT APPLICATION

(21) Application number: **92305524.8**

(22) Date of filing: **16.06.92**

(51) Int. Cl.⁵: **B01D 53/04**, C07C 7/12, C07C 7/13

(30) Priority: **24.06.91 US 701555**

(43) Date of publication of application:
**30.12.92 Bulletin 92/53**

(84) Designated Contracting States:
**BE DE FR GB IT NL SE**

(71) Applicant: **THE BOC GROUP, INC.**
**575 Mountain Avenue**
**Murray Hill New Jersey 07974(US)**

(72) Inventor: **Shirley, Arthur I.**
**373 New Market Road**
**Piscataway, New Jersey 08854(US)**
Inventor: **Ramachandran, Ramakrishnan**
**232 Hillside Avenue**
**Allendale, New Jersey 07401(US)**

(74) Representative: **Gough, Peter et al**
**c/o THE BOC GROUP PLC Patent Department**
**Chertsey Road**
**Windlesham Surrey GU20 6HJ(GB)**

(54) Method of removing gases and light hydrocarbons from gas streams.

㊗ High boiling point gases, i.e. those having boiling points greater than above -80° C., are separated from a gaseous mixture containing, in addition to the high boiling gases, methane and/or a $C_2$ hydrocarbon and one or more gases having boiling points less than -170° C. by subjecting the gaseous mixture to adsorptive separation. If the gaseous mixture contains more than one high boiling component, these components may be further separated by fractional distillation.

FIG. I

This invention relates to the removal of certain components from gas streams, and more particularly to the removal of valuable or environmentally unacceptable components from waste gas streams prior to release of the waste gas streams to the atmosphere.

Waste gas streams from chemical manufacturing or processing plants usually contain light gas elements or compounds which are not incompatible with the environment and thus can be released to the atmosphere. For example, oxygen, nitrogen, argon, water vapor and carbon dioxide are commonly present in significant quantities in waste gas streams. It is also not uncommon for waste gas streams to contain trace amounts of hydrogen and helium. Since these gases are naturally occurring components of the atmosphere, they are environmentally acceptable and may be released to the atmosphere in reasonable quantities.

It often happens, however, that chemical plant waste gas streams contain higher boiling components which cannot be released to the atmosphere in concentrations above a specified tolerance limit. In such cases, the environmentally unacceptable gas components must be removed from the waste gas stream or converted to relatively harmless compounds, such as carbon dioxide, prior to release of the waste gas stream to the atmosphere.

One method of accomplishing the objective of rendering waste gas streams environmentally acceptable prior to releasing them to the atmosphere, is to burn the gases. This method can be employed when substantially all of the harmful components are removed from the gas streams and no other harmful gaseous substance are produced by incineration of the stream. For instance, when the gas stream originally contains only hydrocarbons as heavy gases, the gas stream may be burned, provided that combustion is sufficiently complete that the combustion by-products constitute substantially only water vapor and carbon dioxide.

Combustion of waste gas streams is not always a suitable method for the elimination of environmentally unacceptable components from the stream. For example, when the stream contains significant quantities of hydrocarbon, combustion of the stream will produce large quantities of $CO_2$, the release of which to the environment will additionally burden an already overtaxed ecosystem. Furthermore, it often happens that the gas stream contains components which are not combustible, or which are combustible, but upon burning produce noxious combustion by-products. For example, many chlorinated hydrocarbons are not flammable, and others, although flammable, produce chlorine-containing by-products which themselves are not environmentally acceptable. It can readily be appreciated, therefore, that when a waste gas stream contains components which cannot be safely removed or destroyed from the stream by combustion, other techniques must be employed.

A commonly used technique for removing heavy gas components from waste gas streams is to separate the components by fractional distillation. Thus, high boiling gas components can often be separated from low boiling gases, such as oxygen, nitrogen and argon, by cooling and compressing the gas stream to a temperature below the boiling point of the high boiling components at the existing pressure, and subsequently separating the components by means of distillation. When the gas mixture contains only the light gases mentioned above, i.e., nitrogen, oxygen, argon, etc., and the higher boiling compounds that are to be removed, i.e., substituted hydrocarbons and $C_3$ and higher unsubstituted hydrocarbons, a clean separation of the high boiling gases from the light gases can usually be effected by fractional distillation. However, when methane or any of the $C_2$ hydrocarbons are present in the gas mixture in addition to the low boiling and high boiling gases, it is often difficult to obtain a clean separation by distillation. During the course of the distillation, the $C_1$ and $C_2$ hydrocarbons, because of their low boiling points, distil off with the light gases. However, due to the solubility of the high boiling gas components in the light hydrocarbons, some of the high boiling compounds are also distilled off with the light gases. Thus, conventional fractional distillation cannot be effectively employed to separate high boiling hydrocarbons and substituted hydrocarbons from low boiling gases in gas mixtures when the gas mixtures also contain methane and/or $C_2$ hydrocarbons.

Because of increasingly stringent environmental regulations, and because of the economic advantages offered by recovering and recycling high boiling hydrocarbons, there is a continuing effort to find improved methods for the removal of high boiling hydrocarbons from waste gas streams which additionally contain methane and/or one or more $C_2$ hydrocarbons and low boiling environmentally acceptable gases. The present invention provides an effective and efficient method for accomplishing this result.

According to a first aspect of the present invention, a process for the recovery of gases having boiling points greater than about -80°C. from a gaseous mixture comprising one or more gaseous components having boiling points in the range of about -80°C. to about 50°C., one or more hydrocarbons having boiling points between about -170°C. and about -80°C. and one or more gaseous components having boiling points below about -170°C. is characterised by subjecting said gaseous mixture to adsorptive separation in a bed of adsorbent thereby producing an unadsorbed gaseous product enriched in components having boiling points less than about -80° C. and a desorbed gaseous product enriched in components having boiling points greater than about -80° C.

According to a further aspect of the present invention, a process for the recovery of gases having boiling points greater than about -80° C. from a gaseous mixture comprising one or more heavy gaseous components having boiling points in the range of about -80° C. to about 50° C. one or more hydrocarbons selected from methane, ethane, ethene and ethyne, and one or more light gaseous components selected from oxygen, nitrogen, argon, hydrogen, and carbon monoxide, is characterised by subjecting said gaseous mixture to adsorptive separation in a bed comprising adsorbent selected from activated carbon, silica gel, zeolites, carbon molecular sieves and mixtures of these, thereby producing an unadsorbed gaseous product enriched in said hydrocarbons and light gaseous components and a desorbed gaseous product enriched said heavy gaseous components.

According to yet a further aspect of the present invention apparatus useful for the recovery of gases having boiling points greater than about -80° C. from a gaseous mixture comprising one or more gaseous components having boiling points in the range of about -80° C. to about 50° C., one or more hydrocarbons having boiling points between about -170° C. and about -80° C. and one or more gaseous components having boiling points below about -170° C. is characterised by:

(a) a first adsorption zone containing an adsorbent which adsorbs gases having boiling points greater than about -80° C. more readily than it adsorbs gases having boiling points less than about -80° C;

(b) a second adsorption zone whose inlet is in fluid communication with the product end of said first adsorption zone, and containing an adsorbent which adsorbs hydrocarbons having boiling points in the range of about -170° C. to about -80° C., more readily than it adsorbs gases having boiling points less than about -170° C;

(c) a cryogenic gas separation unit having an inlet in fluid communication with the feed end of said first adsorption zone, and adapted to receive and separate by fractional distillation a multi-component gas stream that is desorbed from said first adsorption zone;

(d) means for introducing said gas mixture into said first adsorption zone at super atmospheric pressures;

(e) means for recovering product gas from said second adsorption zone;

(f) means for recovering desorbed gas from said second adsorption zone; and

(g) means for separately recovering the components separated in the cryogenic gas separation unit.

According to the invention, a gas mixture containing one or more high boiling gases, one or more low boiling gases and one or more light hydrocarbons (all defined below) is subjected to adsorptive separation to produce an unadsorbed product gas stream that is enriched in low boiling gases and light hydrocarbons but substantially free of high boiling gases, and a desorbed gas product stream enriched in high boiling gases. The two product gas streams can then be further treated, if desired, to separate the various components of each of the gas streams.

According to the process aspect of the invention, gases having boiling points greater than about -80° C are separated from a gas mixture containing one or more gaseous components having boiling points in the range of about -80° C to about 50° C, one or more hydrocarbons having boiling points between about -170° C and about -80° C, and one or more gaseous components having boiling points below about -170° C. This is accomplished by passing the gaseous mixture through a bed of adsorbent which adsorbs the gaseous components having boiling points greater than about -80° C more readily than it adsorbs gaseous components having boiling points lower than about -80° C, thereby producing an unadsorbed gaseous product stream which is enriched in those components having boiling points less than about -80° C and a desorbed gaseous product stream that is enriched in components having boiling points greater than about -80° C.

In a preferred embodiment of the process of the invention, the adsorbent is activated carbon, silica gel, zeolites, carbon molecular sieves and mixtures of these.

In another preferred embodiment, the unadsorbed gaseous product stream is subjected to one or more additional adsorptive separations to recover methane and $C_2$ hydrocarbons from the unadsorbed gaseous product stream.

In yet another preferred embodiment, applicable to the separation of gaseous mixtures which contain, in addition to low boiling gases and light hydrocarbons, two or more gaseous components having boiling points in the range of about -80° C to about 50° C, the invention comprises subjecting the high boiling gas-containing desorbed gaseous product stream to one or more fractional distillation steps to separately recover some or all of the components of this stream.

In another preferred embodiment, hydrogen and/or carbon monoxide present in the waste gas stream are oxidised to water and carbon dioxide, respectively subsequent to the adsorption step.

In a most preferred embodiment, the process of the invention is applied to the recovery of two or more gases having boiling points in the range of about -80°C to about 50°C, one or more hydrocarbons selected from methane, ethane, ethene and ethyne, and one or more light gaseous components selected from oxygen, nitrogen, argon, hydrogen and carbon monoxide. According to this aspect of the invention, the gaseous mixture is subjected to a first adsorptive separation in a bed comprising an adsorbent selected from activated carbon, silica gel, zeolites, carbon molecular sieves or mixtures of these, thereby producing an unadsorbed gaseous product stream enriched in low boiling gases and light hydrocarbons, but substantially depleted of high boiling gases, and a desorbed gaseous product stream enriched in high boiling gases. The unadsorbed gaseous product stream is then subjected to a second adsorptive separation, thereby recovering an unadsorbed gas stream enriched in low boiling gases and a desorbed gaseous product enriched in one or more of methane, ethane, ethene and ethyne. Also, according to this embodiment, the desorbed gaseous product enriched in the high boiling gaseous components can be subjected to one or more fractional distillation steps to separately recover the components of the high boiling gas mixture.

Embodiments of the invention will now be described by way of example, reference being made to the Figures of the accompanying diagrammatic drawings in which:-

Figure 1 broadly illustrates, in a schematic diagram, apparatus in which the process of the invention can be carried out.

Figure 2 illustrates, in a schematic diagram, apparatus in which a preferred embodiment of the invention can be carried out.

The invention provides an effective and efficient method for recovering high boiling gases from a gaseous mixture comprised of one or more high boiling gases, one or more low boiling gases and one or more light hydrocarbons. To simplify the description of the invention, these terms are specifically defined as follows:

The term "high boiling gas", is used herein to denote an organic or inorganic gaseous element or compound which, in the liquid state, has a normal boiling point above about -80°C. Examples of high boiling gases are unsubstituted hydrocarbons having three or more carbon atoms, halogenated hydrocarbons, nitrogen-substituted hydrocarbons, oxygen-substituted hydrocarbons and sulphur-substituted hydrocarbons.

The term "low boiling gas" is used to denote a gaseous element or compound which, in the liquid state, has a normal boiling point below about -170°C. Typical of the low boiling gases are the atmospheric gases such as oxygen, nitrogen, argon, helium and hydrogen.

The term "light hydrocarbon" is used to denote a hydrocarbon having a normal boiling point between about -170°C and -80°C. Hydrocarbons having boiling points in this range include methane, ethane, ethene and ethyne.

The "normal boiling point" of an element or a compound is the boiling point of the element or compound at standard pressure, i.e. 760 mm barometric pressure.

According to a main embodiment of the process of the invention, the gas mixture being treated is initially subjected to a high boiling point gas adsorptive separation in an adsorption bed which contains an absorbent that strongly adsorbs high boiling gases, thereby producing an unadsorbed gaseous product that is enriched in low boiling gases and light hydrocarbons but substantially depleted in high boiling gases, and an desorbed gaseous product that is enriched in high boiling gases.

There are a number of options available for treating the unadsorbed gas product stream obtained from the rough cut adsorptive separation step. One option, available when the unadsorbed gas product stream does not contain environmentally objectionable non-atmospheric gas components, e.g. carbon monoxide and light hydrocarbons, at concentrations above their maximum allowable limits, is to vent this stream to the atmosphere.

A second option is to incinerate this stream to burn carbon monoxide, hydrogen and light hydrocarbons. This option is feasible only when the concentrations of these gases in the unadsorbed gaseous product stream are below the concentration levels that would make recovery of these components economically worth while.

A third alternative, worthwhile when the unadsorbed gaseous product stream contains significant concentrations of light hydrocarbons, is to subject this gas stream to a second adsorptive separation to recover the light hydrocarbons from the unadsorbed gas product stream. This is accomplished by introducing the unadsorbed gaseous product stream from the high boiling gas adsorption zone into a second adsorption zone containing an adsorbent that adsorbs the light hydrocarbons contained in the gas stream more strongly than it adsorbs the low boiling gases, thereby producing a second unadsorbed gaseous product stream that is enriched in low boiling gases but depleted in light hydrocarbons and a desorbed gas product stream which is enriched in light hydrocarbons. The unadsorbed gaseous product stream from the light hydrocarbon adsorption section can be discharged to the atmosphere and the desorbed gaseous product stream can be used as fuel or as feed for a chemical process.

The high boiling gas components that were adsorbed in the high boiling gas adsorption section are desorbed during regeneration of the adsorbent. The desorbed high boiling gases can be incinerated if the resulting combustion gases do not contain components that are environmentally objectionable. Alternatively, the high boiling components of this stream may be recovered or otherwise disposed of, if desired. When the desorbed gas stream from the high boiling gas adsorption section contains more than one component this stream may be subjected to one or more fractional distillation steps to recover some or all of the components of the gas stream.

The invention can be better understood from the attached drawings. Only the principal components necessary for practising the invention, together with connecting fluid transfer lines, are included in the drawings. Valves and auxiliary equipment that are not necessary for an understanding of the invention have been omitted.

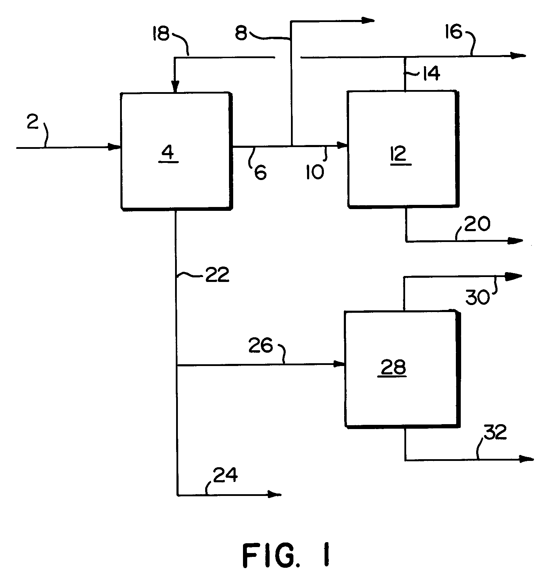

Turning now to the drawings, Figure 1 illustrates, in a schematic diagram, a system in which the process of the invention can be carried out. The major components included in the equipment train shown in Figure 1 are a high boiling point gas adsorption section, 4, a light hydrocarbon adsorption section, 12, and a cryogenic distillation section, 28. Adsorption section 4 is provided with a feed gas line, 2, an unadsorbed gas product line, 6 and a desorbed product gas line, 22. Unadsorbed product gas line 6 joins vent line 8 and hydrocarbon adsorption section feed line 10. A low boiling gas product line 14 exits adsorption section 12 and connects to vent line 16 and rough cut adsorption zone purge gas line 18. Adsorption section 12 is also provided with a desorbed light hydrocarbon product gas line 20. Desorbed high boiling gas line 22 connects high boiling gas adsorption section 4 to both waste gas disposal line 24 and cryogenic distillation section feed line 26. Feed line 26 is, in turn, connected to cryogenic distillation section 28, which is provided with light product discharge line 30 and heavy product discharge line 32.

The high boiling gas adsorption section 4 may comprise a single adsorption unit, but it generally comprises a battery of two or more adsorbers arranged in parallel and operated out of phase with each other so that unadsorbed product gas and desorbed product gas are continuously produced during the operation of this section. In other words while one adsorber of this section is in the adsorption mode another is in the depressurization mode, a third is in the desorption mode etc. Similarly, the light hydrocarbon adsorption section 12 may comprise a single adsorber but generally comprises a battery of two or more adsorption units, likewise operated out of phase to provide a continuous flow of unadsorbed and desorbed product gases. The number, arrangement and design of the adsorbers in each of these adsorption sections is a matter of choice and forms no part of this invention. Similarly, the operation of the adsorption sections in a manner to produce a continuous flow of product streams is well known and likewise forms no part of the present invention.

Fractional distillation section 28 may comprise a single distillation column or a battery of two or more columns arranged in series, depending on the number of components in the unadsorbed product gas stream and the degree of product separation desired. This section may be designed to operate at low temperatures, i.e. under cryogenic conditions, or at higher temperatures or at combinations of these conditions. The design and operation of fractional distillation sections suitable for use in the system of the invention are well known and form no part of the invention.

In practising the process of the invention in the apparatus of Figure 1, a feed gas mixture comprised of one or more high boiling gases, one or more light hydrocarbons and one or more low boiling gases is introduced into adsorber 4 via feed line 2. The high boiling gas components of the mixture are adsorbed onto the adsorbent in adsorber 4, while a large percentage of the low boiling gases and light hydrocarbons entering adsorber 4 pass through the adsorbent bed and exit adsorber 4 through line 6 as the unadsorbed gas product stream. If the unadsorbed gas product stream leaving adsorber 4 contains only trace amounts of light hydrocarbons, this stream may be incinerated or vented to the atmosphere. In this case, these gases exit the system through vent line 8. If, on the other hand, it is desired to subject the unadsorbed gas product stream leaving adsorber 4 to further adsorptive separation, this stream is introduced into adsorber 12 through line 10.

As the unadsorbed gas product stream exiting high boiling gas adsorption section 4 passes through hydrocarbon adsorption section 12, the light gas components of the stream, comprised principally of low boiling gases, pass through the adsorbent bed, exit section 12 through unadsorbed gas product line 14 and leave the system through vent 16. If this gas stream does not contain environmentally objectionably high concentrations of hydrocarbon gases and carbon monoxide it may be vented directly to the atmosphere. If, however, it contains these components in higher than allowable concentrations, this gas stream must be further treated to remove the excess amounts.

The light hydrocarbons entering adsorption section 12 are adsorbed onto the adsorbent. These components are removed from the adsorbent bed during desorption of the bed and are discharged from the system through line 20. As noted above, the desorbed gases can be incinerated or recovered for use in chemical process operations.

The adsorption process conducted in each adsorber of adsorption section 4 is permitted to continue until the high boiling gas front in the adsorber reaches the desired point. At this point, which is prior to breakthrough of the component(s) being adsorbed through the product end of the adsorber in operation, the adsorption step in that adsorber is terminated and the adsorbed gases are desorbed from the bed by depressurization of the adsorber. During the desorption step, the high boiling gases leave adsorber 4 through line 22. Adsorber 4 can be purged with low boiling gas product from adsorber 12 via purge line 18. If the high boiling gases have little value and do not contain components which, upon combustion, result in the production of gas products that are harmful to the atmosphere, they may be discharged to an incinerator through line 24. Likewise, if this gas stream requires additional treatment (other than fractional distillation) or is comprised of a single high boiling component, it may be removed from the system for further processing or storage through line 24.

If the desorbed gas stream in line 22 contains more than one high boiling gas component, the stream components can be separated in fractional distillation unit 28, which, if desired, can be operated under cryogenic conditions. When this option is exercised, the desorbed gas stream enters fractional distillation unit 28 through feed line 26, and the light and heavy product components leave unit 28 through lines 30 and 32, respectively. As indicated above, if the high boiling gas stream contains more than two components, multiple, serially-connected distillation units may be used to effect recovery of the various components of the gas stream.

In some cases the desorbed gas stream from the high boiling gas adsorption section may contain undesirably high concentrations of the low boiling gas components of the gas stream. In such cases, a portion of the desorbed gaseous product stream can be recycled to the feed stream. This will result in a reduction in the overall concentration of low boiling gases in the desorbed product stream.

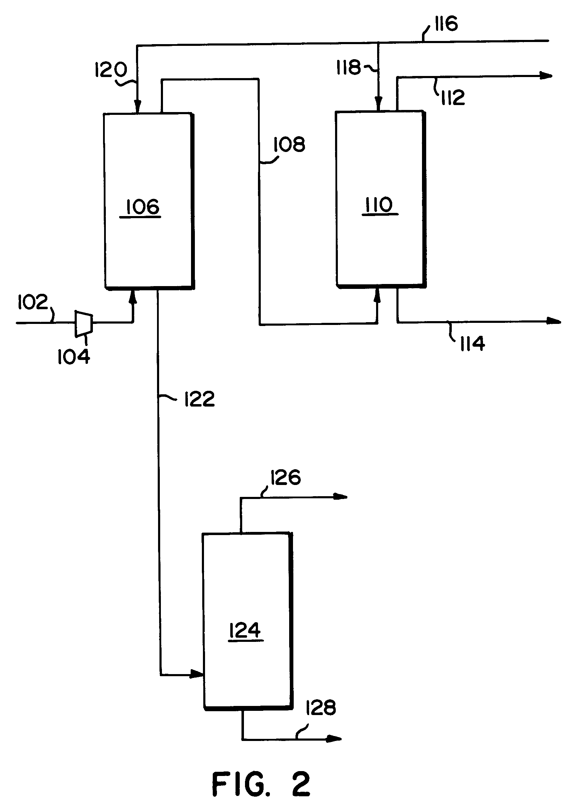

Figure 2 illustrates a simplified version of a preferred embodiment of the invention. The system of Figure 2 is designed for the continuous separation of high boiling gases and light hydrocarbons from low boiling gases contained in a gas stream comprised of these components. The equipment components of the system illustrated in Figure 2 include a feed gas compressor 104, a rough cut adsorption zone 106, a light hydrocarbon adsorption zone 110, and a fractional distillation zone 124.

In practising the process of the invention in the system illustrated in Figure 2, the feed gas mixture, which typically contains the high boiling gases, the low boiling gases and one or more light hydrocarbons selected from methane, ethane, ethene and ethyne, is introduced into the system through feed line 102, is pressurized to the desired pressure in feed gas compressor 104 and is introduced into adsorption zone 106. The high boiling gas components of the mixture are adsorbed onto the bed contained in adsorber 106, while the unadsorbed gas stream, enriched in low boiling gases and light hydrocarbons passes through the adsorption bed and exits the adsorption zone through product line 108. The unadsorbed gas stream next enters light hydrocarbon adsorption zone 110 wherein the $C_2$ hydrocarbons and perhaps some or all of the methane contained in the gas stream are adsorbed onto the adsorbent in adsorber 110 and the unadsorbed gas stream, enriched in low boiling gases, exits the adsorber and leaves the system through line 112.

When the beds in the adsorption modes in zones 106 and 110 become saturated with adsorbed gas, the beds are switched in the well known manner and the saturated beds are then regenerated, thereby producing the desorbed gas streams. During the regeneration modes it may be desirable to purge the beds with the unadsorbed product gas from adsorption zone 110, which is comprised substantially of low boiling gases. This can be accomplished by passing the low boiling gas stream produced in adsorption zone 110 through lines 116, 118 and 120 and then countercurrently through adsorption zones 106 and 110.

The desorbed gas stream leaving adsorption zone 110, comprised predominantly of light hydrocarbons, can be incinerated or used in other chemical processes.

The desorbed gas stream leaving adsorption zone 106 during desorption of the adsorbers in this zone is comprised predominantly of high boiling gases. This stream passes through line 122 and enters fractional distillation unit 124, which separates the gas components into a light gas product and a heavy gas product. The light gas product leaves unit 124 through line 126 and the heavy product leaves unit 124 through line 128. If the high boiling gas stream contains more than two components these may be separately recovered by means of additional fractional distillation units arranged in series with unit 124.

The invention is further illustrated in the following hypothetical example wherein, unless otherwise indicated, parts, percentages and ratios are on a volume basis.

EXAMPLE 1

A gas stream having the composition indicated in TABLE 1 is treated by the process of the invention in a simulated run to remove methyl chloride from the stream. The equipment train used in the simulated run is similar to the equipment train illustrated in Figure 2 and comprises a first set of adsorbers containing silica gel and a second set of adsorbers containing activated carbon. The feed stream entering each set of adsorbers is at a pressure in the range of about 20 - 25 psia. The gases adsorbed in each set of adsorbers is desorbed at an absolute pressure of about 200 millibars. About 61% of the desorbed gas stream from the first set of adsorbers is recycled to the feed stream. The remainder of the desorbed gas stream from the first set of adsorbers is discharged from the system as desorbed product. The projected molar concentrations of the components in each stream is tabulated in the TABLE.

**TABLE**

| Component | Feed to First Adsorbers | Nonadsorbed Prod., First Adsorbers | Desorbed Product, First Adsorbers | Nonadsorbed Prod., Second Adsorbers | Desorbed Product, Second Adsorbers |
|---|---|---|---|---|---|
| Methane | 21.6 | 15.1 | 6.5 | 0.1 | 15.0 |
| Ethylene | 4.4 | 1.1 | 3.3 | 0.0 | 1.1 |
| Nitrogen | 20.6 | 17.6 | 3.0 | 16.9 | 0.7 |
| Methyl Chloride | 2.5 | 0.0 | 2.5 | 0.0 | 0.0 |
| TOTAL | 49.1 | 33.8 | 15.3 | 17.0 | 16.8 |

The above example illustrates the projected efficiency of the invention for removing high boiling gaseous components from a gas stream. In the process depicted in EXAMPLE 1, all of the methyl chloride in the feed stream will be adsorbed in the first set of adsorbers, while the non-adsorbed product from the second set of adsorbers will contain 16.9 moles of nitrogen (about 85.4% of the nitrogen in the feed stream).

Although the invention has been described with particular reference to the illustrated embodiments and a specific example, variations of these are contemplated. For example, other gas separation techniques, such as membrane separation and absorption can be used in the invention in combination with the adsorption steps depicted in the drawings. Furthermore, the efficiency of the disclosed process can be increased by conducting multiple adsorption steps in series and/or in parallel and by recycling any of the product streams from the various adsorbers. The scope of the invention is limited only by the breadth of the appended claims.

## Claims

1. A process for the recovery of gases having boiling points greater than about -80° C. from a gaseous mixture comprising one or more gaseous components having boiling points in the range of about -80° C. to about 50° C., one or more hydrocarbons having boiling points between about -170° C. and about -80° C. and one or more gaseous components having boiling points below about -170° C. characterised by subjecting said gaseous mixture to adsorptive separation in a bed of adsorbent thereby producing an unadsorbed gaseous product enriched in components having boiling points less than about -80° C. and a desorbed gaseous product enriched in components having boiling points greater than about -80° C.

2. The process of Claim 1, characterised in that said gaseous mixture contains two or more gaseous components having boiling points in the range of about -80° C. to about 50° C.

3. The process of Claim 2, characterised in that said two or more gaseous components having boiling points in the range of about -80° C. to about 50° C. are further separated by fractional distillation subsequent to desorption from said adsorbent.

4. The process of Claim 1, 2 or 3, characterised in that the gaseous components present in said gaseous mixture having boiling points in the range of about -80° C. to about 50° C. include one or more members selected from the group consisting of hydrocarbons having three or more carbon atoms, halogenated hydrocarbons, nitrogen-substituted hydrocarbons, oxygen-substituted hydrocarbons and sulfur-substituted hydrocarbons.

5. The process of Claims 1 to 4, characterised in that said gaseous mixture contains one or more components selected from the group consisting of oxygen, nitrogen, argon, hydrogen and carbon monoxide.

6. The process of Claims 1 to 5, characterised in that hydrogen is present in said gaseous mixture and it is oxidized to water subsequent to the adsorption step.

7. The process of Claims 1 to 6, characterised in that carbon monoxide is present in said gaseous mixture and it is oxidized to carbon dioxide subsequent to the adsorption step.

8. The process of Claim 1, characterised in that at least one of said gaseous hydrocarbons is separated from said unadsorbed gaseous product in a second adsorptive separation step.

9. A process for the recovery of gases having boiling points greater than about -80° C. from a gaseous mixture comprising one or more heavy gaseous components having boiling points in the range of about -80° C. to about 50° C. one or more hydrocarbons selected from methane, ethane, ethene and ethyne, and one or more light gaseous components selected from oxygen, nitrogen, argon, hydrogen, and carbon monoxide, characterised by subjecting said gaseous mixture to adsorptive separation in a bed comprising adsorbent selected from activated carbon, silica gel, zeolites, carbon molecular sieves and mixtures of these, thereby producing an unadsorbed gaseous product enriched in said hydrocarbons and light gaseous components and a desorbed gaseous product enriched said heavy gaseous components.

10. The process of Claim 9, characterised in that said unadsorbed gaseous product is subjected to a second adsorptive separation thereby producing a light product phase enriched in said light gas components and a desorbed phase enriched in one or more of methane, ethane, ethene and ethyne.

11. Apparatus useful for the recovery of gases having boiling points greater than about -80° C. from a gaseous mixture comprising one or more gaseous components having boiling points in the range of about -80° C. to about 50° C., one or more hydrocarbons having boiling points between about -170° C. and about -80° C. and one or more gaseous components having boiling points below about -170° C. characterised by:

(a) a first adsorption (106) zone containing an adsorbent which adsorbs gases having boiling points greater than about -80° C. more readily than it adsorbs gases having boiling points less than about -80° C;

(b) a second adsorption zone (110) whose inlet is in fluid communication with the product end of said first adsorption zone (106), and containing an adsorbent which adsorbs hydrocarbons having boiling points in the range of about -170° C. to about -80° C., more readily than it adsorbs gases having boiling points less than about -170° C;

(c) a cryogenic gas separation unit (124) having an inlet in fluid communication with the feed end of said first adsorption zone (106), and adapted to receive and separate by fractional distillation a multi-component gas stream that is desorbed from said first adsorption zone;

(d) means (104) for introducing said gas mixture into said first adsorption zone (106) at super atmospheric pressures;

(e) means for recovering product gas from said second adsorption zone (110);

(f) means for recovering desorbed gas from said second adsorption zone (110); and

(g) means for separately recovering the components separated in the cryogenic gas separation unit (124).

12. The apparatus of Claim 11, characterised by means (120) for purging said first adsorption zone (106) with product gas from said second adsorption zone.

FIG. 1

FIG. 2